# EUROPEAN PATENT SPECIFICATION

(11) **EP 1 281 349 B1**
(45) Date of publication and mention of the grant of the patent: **25.01.2006**
(21) Application number: 02016487.7
(22) Date of filing: 23.07.2002
(51) Int. Cl.: A61B 1/31, A61B 8/06

(54) **Retractor for surgical operations on the arteria haemorrhoidalis**
Retraktor für Operationen der Arteria haemorrhoidalis
Ecarteur pour des opérations chirurgicales sur l'artère hémorroidale

(30) Priority: 02.08.2001 IT BO20010502
(43) Date of publication of application: 05.02.2003
(73) Proprietor: Metech S.r.l., 42015 Corregiio (Reggio Emilia) (IT)
(72) Inventor: Burgoni, Roberto, 40138 Bologna (IT)
(74) Representative: Gotra, Stefano

(56) References cited:
- DE-A- 19 929 314
- DE-C- 830 544
- FR-A- 2 623 078
- IT-B- 1 234 169
- US-A- 4 766 886
- US-A- 5 570 692

## Description

In order to effect ambulatory operations on the haemorrhoids, without anaesthesia, it is known the use of the device described in the U.S. patent No. 5 570 692, which comprises a retractor tube closed on the end which is inserted in the anal cavity and opened on the external end, which is provided with a gripping handle. The retractor tube is provided on its lateral wall, at a short distance from its closed end, with an ultrasonic probe to detect the blood flow of the haemorrhoidalis artery and is provided near to the probe, with a lateral window through which may be detected and observed the portion of the anal mucosa upon which it must be operated for the ligature of said artery, for example by means of a curved needle or by means of cauterisation. The closed end of the retractor tube, may be illuminated by a luminous source housed in said end and connected to feeding means provided in the handle, together with the feeding means of said probe. This device, for the reason that incorporates the ultrasound probe and that houses the luminous source in its closed end, presents elevated production costs, so that it is not possible to propose the same as a disposable product, with all the drawbacks and the limitations deriving by this fact.

Object of the invention is to obviate to these and other limits of the known prior art, with a disposable device, for the realisation of which it has been necessary to resolve some technical problems connected with the removable housing in the same of the ultrasonic probe and other problems related to the means for the illumination of the lateral window for the exploration of the anal mucosa. The first of said problems has been solved providing in the retractor tube a longitudinal seat, closed toward the interior and opened with the end toward the outer end of the same tube, in which seat it is possible to removably house an ultrasonic probe which partially projects through a longitudinal opening of the retractor tube, to result in contact with the anal mucosa. The ultrasonic probe is preferably inserted and hygienically protected in a sterile, disposable and easily removable sheath, having a suitable conformation, in such a manner that the same may be reutilized repeatedly in other disposable devices of the type which is referred to. Immediately downstream of the seat with the ultrasonic probe, there is provided the window for the exploration of the anal mucosa. To solve the problem of the illumination, it has been used the technique of the back illumination, known in the proctoscopies, which provides the movable insertion of a luminous source in the handle of the device. Instead of the use of curved light guide means, realised for example with optical fibre or with a bar of plastics, connected with one of their end to said luminous source and oriented with the other end in the field of view defined by the internal cavity of the retractor tube, as described for example in the Italian patent No. 1 234 169, in the device according to the invention are utilised curved means to reflect the light inside of the retractor tube, with the advantage of a better luminous yield and with the advantage that such devices result distant from the internal surface of the same retractor and can not be soiled and blinded by the physiological liquid which unavoidably is produced by the anal cavity during the operation which is referred to.

These and other features of the invention, and the advantages deriving therefrom, will appear better evident from the following description of a preferred embodiment of the same, made by way of non-limiting example, with reference to the figures of the attached sheets of drawings, in which:

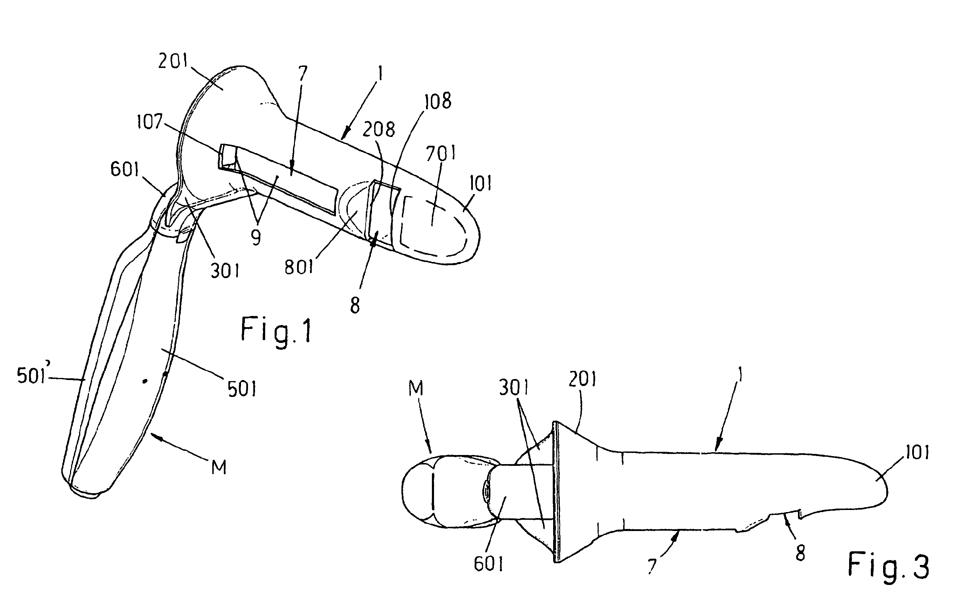

- Figure 1 is a perspective view of the device without the ultrasonic probe and without the illumination source;
- Figure 2 shows the device in lateral elevation, decomposed in the pieces which compose it and with the illumination source placed between the two portion of the handle in which the same is inserted;
- Figures 3 and 4 show the device respectively in plan view from above and in plan view from the bottom;
- Figure 5 shows the device assembled and sectioned along the line V - V of Figure 2;
- Figure 6 shows further details of the device sectioned along the line VI - VI of Figure 5;

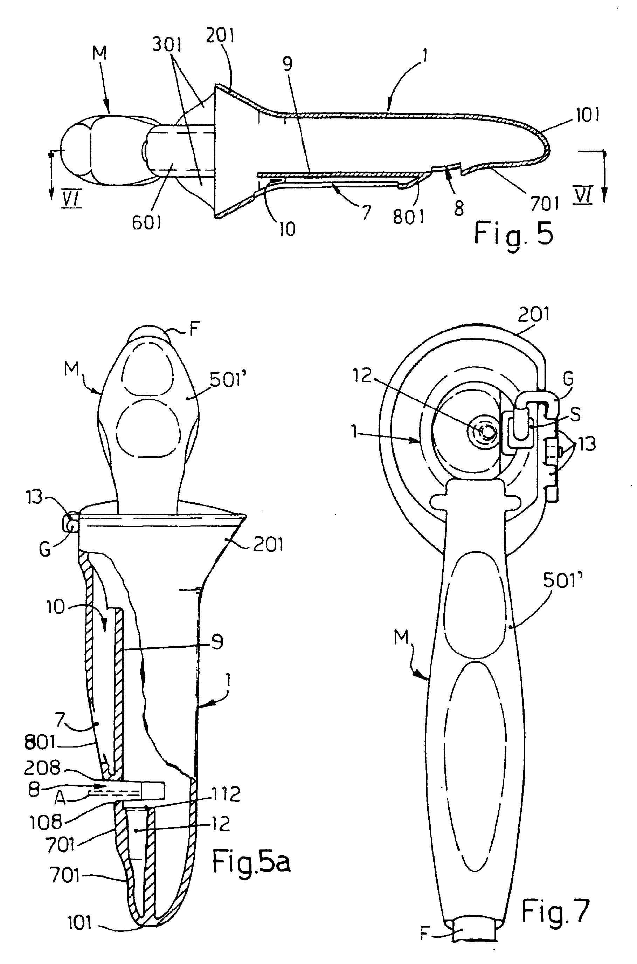

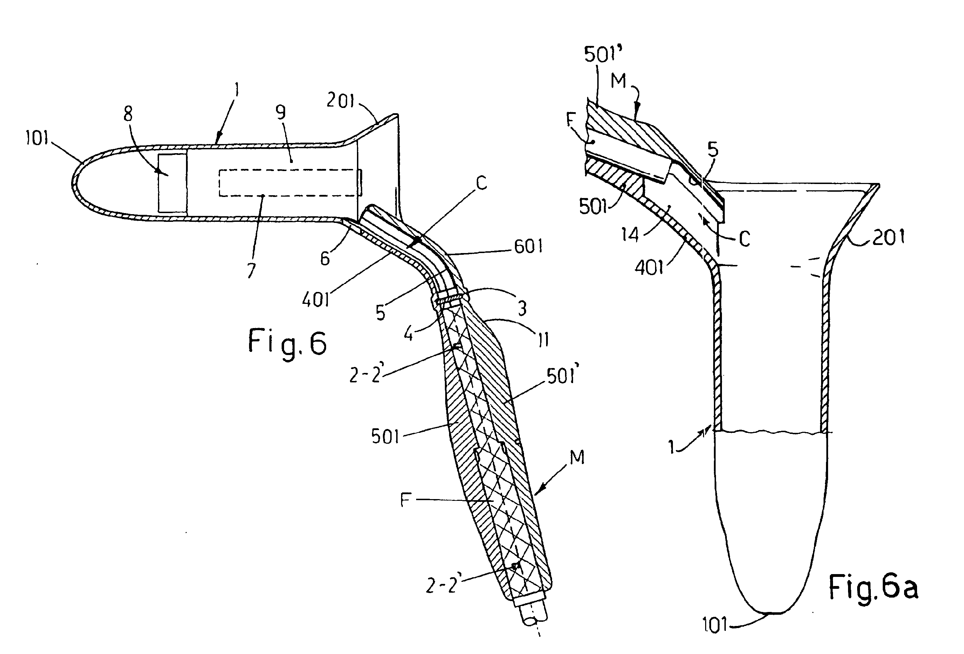

- Figures 5a and 6a show embodiments of the device respectively viewed as in the preceding Figures 5 and 6;
- Figure 7 shows the device of the Figure 5a according to a view of the front toward the operator.

From the Figures it is noted that the device comprises a substantially cylindrical tube 1 having the function of retractor, closed at its terminal end 101 which is opportunely tapered and rounded, and on the contrary open on the initial end 201 which has a conical shape and which is outwardly divergent. Merely by way of example, the body 1 may have an external diameter which is comprised between 2,5 and 3,5 centimetres, for example of about 3 centimetres, an may have a general length comprised between 10 and 12 centimetres, comprehensive of the divergent end 201 which has alone the length of about 2 centimetres. However, it is to be understood that the device may be realised with dimensions which are different to the indicated dimensions, in order to comply with different use requirements. The conical end 201 is outwardly projecting with a portion having a substantially triangular plan 301, having a length of some centimetres, provided in its center line with a longitudinal and channel-shaped rib 401, outwardly convex, which has prevalently the function to increase the resistance to the bending and torsion stress of said appendices 301 and to partially define the duct along which will be effected the reflection of the light for the illumination of the working zone. The appendix 301, which has for example an inclination of about 30° with respect to the longitudinal axis of the body 1, integrally connects to said body an elongated shell 501 realised with a suitable ergonomic shape for the function of gripping handle, having for example a length of about 10 centimetres and which is forming with the axis of said body 1 an internal angle of about 105°. It is to be understood that also these last dimension data of the device are merely indicative and that the same may be widely modified. The connecting zone of the shell 501 to the appendices 301, is suitably curved. Upon the shell 501 there is placed and fixed with the male-female fixed coupling portions 2, 2', a complementary shell 501' which completes the formation of the gripping handle M and which is superimposed to the ribbed portion 401 of the appendix 301, with a terminal portion 601 having the shape of a channel, which is connected to the same shell 501' with a suitable curvature, which ends in the connecting zone of the conical portion 201 to the cylindrical portion of the tube 1 and then in the internal portion of said tube which appears through the same conic mouth 201. The portion 601 realises with the portion 401 a tubular duct C which at least from the outside presents a flat shape also for the presence of lateral ribs, in such a manner to result with high features of resistance to the bending and to the torsion. In the conjunction zone of the shell 501 to the channel 601, there is provided a step 11 raised toward said shell, suitable for leaning the thumb of the hand which grasps the handle M, to ensure a steady grasp of the same handle and to facilitate in absolute the use of the device.

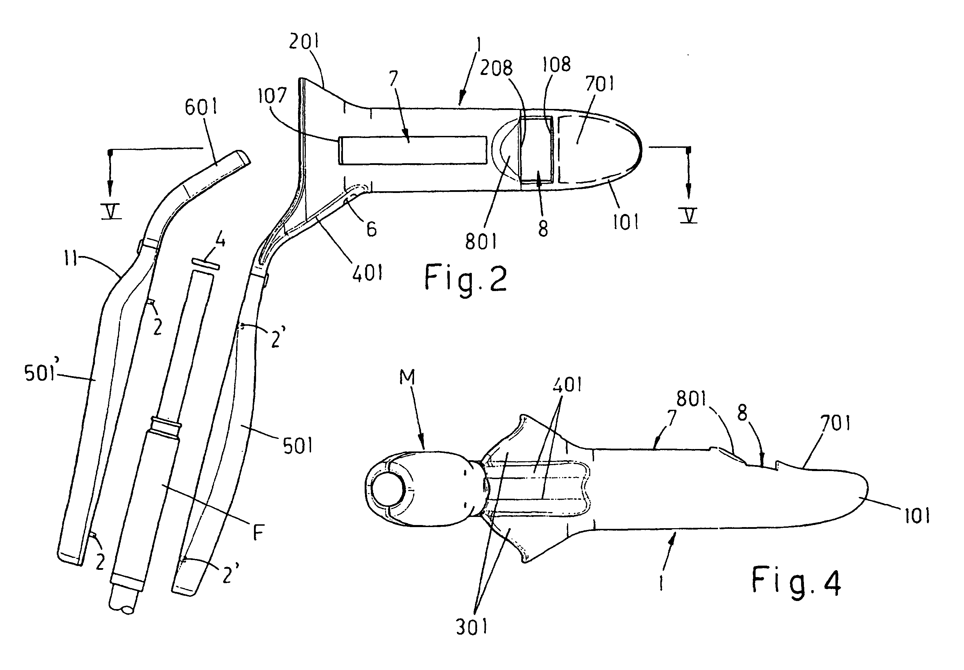

In the conjunction zone of the unit of the handle M to the appendix 301, inside of the two complementary shells which define the same handle, are obtained the two complementary portions of an annular seat 3 in which can be placed a small disk 4 made of transparent material, which realises a division barrier between the internal and absolutely sterile portion of the instrument, from the internal and hollow portion of the handle, in which is inserted and retained for example by means of friction, the end of the illumination optical waveguide F, of the known type, which may be not subjected to sterilisation treatments. The small disk 4 may have, if required, optical functions and may be made by means of a lens suitable to focalise the light on a reflection parabola 5 which covers the internal surface of the portion 601 of the duct C and which can be, for example, realised in a very economical and reliable manner, with an electrochemical metal spray coating of chrome. The advantages deriving from the backlight system described, with respect to the known systems which use light guides, are represented by a better luminous efficiency and especially by the fact that the same illumination means may be not blinded by the organic liquid which can come out from the hollow of the retractor tube, because the reflection parabola 5 remains raised from the path of said liquid, and because also in the most unfavourable condition shown in Figure 6, it is possible to foresee upstream of the small disk 3, on the ribbed zone 401, one or more drainage openings 6, suitably shaped, through which said organic liquid may freely come out. The device is preferably realised with plastics of a changing white colour, to exalt the effects of the illumination inside the body 1. From the drawings, it appears that the retractor tube 1 is laterally provided with a longitudinal and rectilinear opening 7, for example with a rectangular shape, which begins in the zone in which the end conic portion 201 is connected to the cylindrical portion of the same retractor 1 and which has a length which is about equal to the half length of the same retractor. In the example which is referred to, the ideal plane in which lies the opening 7 is parallel to the center line plane of the device and the same opening is placed on the right side of the body 1 if the device is considered with the handle M downwardly oriented, but it is to be understood that said collocation may be divorsified. It is not even excluded that the ideal plane on which the opening 7 lies, may be differently perpendicular to the vertical center line plane of the device, with the same window which results placed in the upper portion of the body 1 if the device is considered with the handle downwardly oriented, also to cause the terminal and internal portion of the body retractor, placed downstream of the zone interested by said opening, may be better illuminated by the beam which comes out from the reflection parabola 5. In fact, in said zone, the retractor body 1 presents a tapered and slightly flattened shape, as shown with numeral reference 701, in the initial portion of which is provided, transversally oriented with the greatest dimension, a window 8 for example with a rectangular shape, for example having the dimensions of centimetres 1 x 2, through which it will appears the anal mucosa which will be efficaciously illuminated by the above mentioned backlight means. The window 8 is distant from the outer end of the body 1, which is connected to the conical portion 201, of about 4-7 centimetres, for example of about 5-6 centimetres. In the zone which is comprised between the rear edge 208 of the window 8 and the rear side 107 of the openings 7, the body 1 presents internally and integral a flat division wall 9, which delimits inside the same body 1 a longitudinal chamber 10 open on the end toward the mouth 201 of the retractor and provided with the outer and lateral opening 7 above mentioned. In said chamber 10 is friction inserted a ultrasound probe, not illustrated, which will be realised with such shape to opportunely project from the opening 7, to result in contact with the rectal mucosa. As said in the introduction of the present description, the probe may be contained in a thin sterilised, disposable and easily removable sheath, so that the same probe may be used several times in other disposable devices of the type which is referred to. The connection cable to the probe, will go out from the mouth 201 of the retractor and it may be temporarily fixed with an adhesive bandage on a side of the handle M. It is to be understood that the handle M and other portions of the device (see further) may be laterally provided with small loops having the shape of pincers, integral obtained upon the shells 501, 501' and suitable to temporarily support the cable of the ultrasound probe above mentioned. As appears from Figures 3-5, the window 8 lies on a terminal portion of the retractor tube which is slightly flattened and in recess and the rear side 208 of said window is connected with an inclined plane 801 with the lateral surface of the retractor. The forward edge 108 of the window 8 is then characterised by the fact that it is in relief and to have a slightly arcuate shape, with the convexity turned toward the outside. All these conditions allow to optimise the dilatation of the rectal tissues and contextually to avoid prolapse of the same inside the window 8, in such a manner that through said window the rectal mucosa presents itself in the better condition to operate on the same with the known and required means for the ligature of the arteria haemorrhoidalis, which can be identified with precision by means of the said ultrasonic probe.

The device shown in Figures 5a, 6a and 7 is different from the device previously described for the several features below considered. The window 8 is, for example, arcuate-shaped, is obtained on the retractor tube 1 substantially for half of its circumference, and has a length which is inferior to 1 centimetre, for example comprises between 8 and 5 millimetres. The inclined plane 801, placed immediately downstream of the window 8, is more wide and less inclined of that of the Figures from 1 to 5, and upon it there is localised the opening 7 which exposes the sensible portion of the ultrasonic probe S visible in Figure 7, in such a manner that this same portion results very close to the said window 8 and to the portion of the arteria haemorroidalis upon which the operation will be made.

The forward side 108 of the window 8 it is not in relief as in the previous solution, but it is lower with respect to the posterior side 208 of the same window and forms part of a flat portion 701' which is substantially aligned to the wall 9 for the delimitation of the chamber 10 housing the probe S, said portion being connected with a correct union to the remaining flat portion 701, in such a manner to form in the whole a flat portion with a sinuous profile and with a decreasing profile toward the rounded point 101.

Always from Figure 5a it appears that under the portions 701, 701' above mentioned, inside the body 1 is obtained a seat 12 having for example a rounded section and a conic shape, with a superior edge 112 slightly placed beyond the anterior edge 108 of the window 8, in such a manner to rest upon said edge and to insert in said seat, the terminal portion of a mandrel not shown, which holds the curved needle A with which will be made the ligature of the arteria haemorroidalis and that with the external end of the retractor tube 1 may be easily operated by the operator. The axis of the seat 12 is for example parallel and suitably displaced from the axis of the retractor tube 1.

From Figures 5a and 7 it is noted that the initial conic portion 201 of the retractor 1, is flattened on the side corresponding to the seat 10 for the housing of the probe S and on this side it carries a set of three appendices 13 upon which it is possible to firmly anchor the portion of the cable G which is near to the same probe.

From Figure 6 it is finally noted that the reflecting portion 5 is placed only in the terminal and rectilinear portion of the channel C, with an inclination of about 40-45° with respect to the longitudinal axis of the retractor 1, for example of about 43°. The terminal portion of the optical waveguide F for the illumination is now placed at a short distance from the reflecting surface 5, in such a manner to sensibly improve the illumination intensity of the internal cavity of the same retractor. The longitudinal axis of the terminal portion of the optical waveguide F inserted in the handle M, forms with the axis of the retractor 1 an internal angle of about 110°. Always from Figure 6a it is finally noted how the same terminal portion of the optical waveguide F results raised from the bottom of the channel C with the reflecting surface 5, for the presence of the wide recessed portion 14 in the conjunction zone 401 of the handle M to the retractor tube 1, zone which may be provided with, if required, said drainage opening/s.

## Claims

1. Disposable device for surgical operations on the arteria haemorroidalis, comprising a retractor tube (1) closed and rounded at the end with which to be inserted into the anal orifice, which is provided upon its side surface with at least a window (8) through which there appears the rectal mucosa upon which the operation for the ligature of the artery will be made, which is provided with a gripping handle (M) integral with its external mouth and preferably diverging (201), through which it is possible to observe the tissue through said window and it is possible to insert in the retractor tube (1) the instruments required for the surgical operation, comprising means to illuminate said window and the mucosa which appears through this latter, **characterised in that** said illumination means comprise a luminous source (F) fixed in removable manner inside said gripping handle (M) and comprise at the level of the conjunction zone of said gripping handle (M) to the external end of the retractor tube (1) means (5) to reflect the light supplied from said luminous source (F) and to allow that said reflected light illuminates the internal portion of the retractor tube (1) and particularly, said lateral window (8): the retractor tube (1) being provided in alignment with the window (8) and, upstream of this, with a small longitudinal, rectilinear and internal chamber (10), delimited by a baffle (9) which is integral with the internal walls of the retractor tube (1) and with the posterior side (208) of the window (8), the whole in such a manner that the chamber (10) results opened toward the mouth (201) of said retractor tube (1) there being provided that in said chamber (10) is friction-housed an ultrasonic probe (S) which is partially projecting through an opening (7) longitudinally obtained on the lateral wall of the retractor tube (1) in such a manner that the same ultrasonic probe (S) results to be in contact with the anal mucosa.

2. Device according to claim 1, **characterised in that** the means which provide to reflect the light supplied by the luminous source (F), comprise a specular parabola (5) longitudinally placed and in the portion raised from the bottom of a tubular channel (C) which connects the hollow portion of the handle (M) which houses said luminous source (F), to the external end of the retractor tube and which terminates at the interior of said tube.

3. Device according to claim 2, in which the tubular channel (C) which houses the reflection parabola (5) of the light, has at least externally a flat configuration which derives from a suitable reinforcement of the sides, in such a manner to result very resistant to bending and torsion.

4. Device according to claim 2, in which at least the high portion (601) of the channel (C) which houses the reflection parabola (5) is inserted and fixed upon the other portion of the same channel with the purpose to simplify the realisation and/or the installation of said reflecting parabola.

5. Device according to claim 4, in which the reflecting parabola (5) is realised with an electrochemical process of chromium plating of the internal surface of the high and inserted portion (601) of the channel (C).

6. Device according to claim 4, in which the high and inserted portion (601) of the channel (C) with the light reflecting parabola (5) is integrally obtained and with a connection opportunely jointed, with the upper shell-shaped portion (501') of the gripping handle (M), said portion being predisposed for example for the snap-coupling, by means of male-female appendices (2, 2') with the complementary inferior portion also shell-shaped (501) which completes the same handle (M) and which is integral with the longitudinally ribbed and outwardly convex portion (401) which forms the lower portion of the same channel (C) and which is obtained in an appendix (301) having a substantially triangular plan, with the sides which are tangent to the external edge of the conic mouth (201) of the retractor tube.

7. Device according to claim 6, in which the connecting zone of the upper portion (601) of the channel (C) with the internal light reflection parabola (5), to the upper shell (501') for the formation of the handle (M) presents a step (11) raised toward the said handle, upon which the operator can advantageously lean the thumb of the hand which grasps the same handle, to ensure a more steady grasp and a more easy use of the device.

8. Device according to claim 6, in which in the portion with which the two shells (501, 501') for the formation of the handle (M) are connected with the portions (601, 401) of the channel (C) which contains the light reflecting parabola (5) upstream of said channel, in said shells are obtained complementary portions for the formation of a seat (3) in which it is possible to house a small disk (4) of transparent material, which divides as much as possible in a tight manner said channel (C) from the seat of the handle in which is friction-fixed or in another removable manner the illumination source (F).

9. Device according to claim 8, in which the transparent small disk (4) may have optical functions, for example may be made by a lens which focalises on the reflection parabola (5) the light supplied by the luminous source F.

10. Device according to the preceding claims, in which the channel (C) with the light reflection parabola (5), presents an inclination comprised between 30° and 50°, for example 43°, with respect to the axis of the retractor tube (1), while the internal angle existing between the handle (M) and the axis of the retractor tube (1) is comprised between 100° and 120° and is for example of about 110°.

11. Device according to the preceding claims, **characterised by** the fact that it is formed with any suitable plastics of changing white colour, which facilitates the internal illumination of the retractor tube (1) and particularly of its lateral window (8).

12. Device according to one or more of claims 2-11 **characterised by** the fact that on the inferior portion (401) of the channel (C) which contains the light reflecting parabola (5) there may be provided at least one opening (6) to discharge outwardly the possible organic liquid which arrives by gravity to said opening and to avoid that such liquid arrives on the small disk (4) placed upwardly of the luminous source (F).

13. Device according to one or more of claims 2-12 in which the channel (C) which contains the light reflection parabola (5), presents in contrast to the same parabola, a wide recess zone (14) which leaves uncovered a portion of the end of the optical illumination fiber (F) which is placed at a short distance from the said parabola.

14. Device according to claim 1, in which the window (8) for the exploration of the anal mucosa, lies on an ideal plane which is substantially parallel to the center line plane of the same device and may be placed on the right or on the left of the retractor probe, if the same device is considered with the handle (M) downwardly oriented.

15. Device according to claim 1, in which the window (8) for the exploration of the anal mucosa, lies on an ideal plane which is substantially perpendicular to the center line plane of the same device and is placed upwardly if the same device is considered with the handle (M) downwardly oriented.

16. Device according to claim 1, in which the window (8) for the exploration of the anal mucosa has a distance from the end of the retractor tube which is connected to the conic end (201), which is comprised between 4 and 7 centimetres, for example of about 5-6 centimetres.

17. Device according to claim 1, in which the window (8) for the exploration of the anal mucosa, lies on a flattened and in slight recess portion (701) of the lateral wall of the retractor tube (1) which is near to the rounded and closed end (101) of said tube, the rear side (208) of said window being rounded and connected with an inclined plane (801) with the lateral surface of said tube, while the forward side (108) of the same window is also rounded and suitably raised and presents an arcuate shape, the whole for a better disposition of the anal tissue for the exploration and for the operation through said window.

18. Device according to claim 1, in which the window (8) for the exploration of the anal mucosa is transversally obtained on the retractor tube and interests the same for about the half of its circumference, the rear side (208) of said window being rounded and connected by means of a wide inclined plane (801) with the lateral surface of said tube, while the forward side (108) of the same window it is rounded too, is opportunely lowered with respect to the said rear side and it is connected to flat portions (701', 701) which extend with a sinuous shape and with a decreasing profile toward the rounded point (101) of the retractor tube.

19. Device according to daim 1 **characterised by** the fact that inside of the retractor tube (1) under the lateral window (8) for the exploration of the anal mucosa, at a short distance and preferably at the level of its center line there are provided means (12) suitable to receive and to rotatably center the end of a mandrel which carries the curved needle for the ligature of the arteria haemorroidalis.

20. Device according to claim 19, in which said receiving and centring means (12) are constituted by a rounded section seat, placed with its axis parallel and with a correct distance from the axis of the retractor tube (1).

21. Device according to claim 20 in which said seat (12) has a conical shape and gets narrower toward the point of the retractor tube (1).

22. Device according to claim 1 in which the lateral opening (7) through which is projecting the sensible portion of the ultrasonic probe (S) interests the inclined portion (801) which is converging on the posterior wall (208) of the window (8) for the exploration of the anal mucosa.

23. Device according to claim 1 in which the ultrasonic probe (S) may be hygienically protected in a sterile, disposable and easily removable sheat, to allow hygienic re-utilisation of the same probe.

24. Device according to one or more of the preceding claims, in which the side of the conic mouth (201) of the retractor tube is flattened on the side of the seat (10) for the housing of the ultrasonic probe (S) and carries appendices (13) having the function of loops to which it is possible to removably anchor the cable (G) of the same probe.

25. Device according to claim 24, in which the handle (M) may be provided on the side near to the ultrasonic probe (S) with appendices which realise small loops to which may be removably anchored a further portion of the multipolar cable connected to the said ultrasonic probe (S).

## Patentansprüche

1. Vorrichtung für chirurgische Eingriffe an der Arteria haemorrhoidalis, enthaltend ein Retraktorrohr (1), geschlossen und abgerundet an dem Ende, mit dem es in die Analöffnung eingeführt wird, und welches an seiner Seitenfläche mit wenigstens einem Fenster (8) versehen ist, durch das die Rektalschleimhaut erscheint, an welcher der Eingriff für die Ligatur der Arterie vorgenommen wird, und welche mit einem Handgriff (M) versehen ist, in einem Stück mit der äusseren Öffnung und vorzugsweise divergierend (201), durch welche es möglich ist, das Gewebe durch das genannte Fenster zu beobachten, und es weiter möglich ist, in das genannte Retraktorrohr (1) die für den chirurgischen Eingriff erforderlichen Instrumente einzuführen, einschliesslich den Mitteln zum Beleuchten des genannten Fensters und der Schleimhaut, die durch letzteres sichtbar wird, **dadurch gekennzeichnet, dass** die genannten Beleuchtungsmittel eine Leuchtquelle (F) enthalten, die auf lösbare Weise im Inneren des genannten Handgriffs (M) befestigt ist, und auf der Ebene des Anschlussbereichs des genannten Handgriffes (M) an das äussere Ende des Retraktorrohres (1) Mittel (5) enthalten, um das von der genannten Leuchtquelle (F) erzeugte Licht zu reflektieren und zu erlauben, dass das genannte reflektierte Licht den internen Abschnitt des Retraktorrohres (1), und besonders das genannte seitliche Fenster (8) beleuchtet; wobei das Retraktorrohr (1), ausgerichtet zu dem Fenster (8) und stromaufwärts desselben, mit einer kleinen länglichen, rechteckigen und internen Kammer (10) versehen ist, abgegrenzt durch eine Wand (9), welche in einem Stück mit den internen Wänden des Retraktorrohres (1) und mit der hinteren Seite (208) des Fensters (8) ist, und zwar insgesamt auf solche Weise, dass die Kammer (10) sich in Richtung der Öffnung (201) des genannten Retraktorrohres (1) als offen erweist, wobei vorgesehen ist, dass in der genannten Kammer (10), gehalten durch Reibung, eine Ultraschallsonde (S) angeordnet ist, welche teilweise durch eine Öffnung (7) ragt, die in Längsrichtung in die Seitenwand des Retraktorrohres (1) auf solche Weise eingearbeitet ist, dass dieselbe Ultraschallsonde (S) mit der Analschleimhaut in Kontakt kommt.

2. Vorrichtung nach Patentanspruch 1, **dadurch gekennzeichnet, dass** die Mittel, welche das durch die Leuchtquelle (F) erzeugte Licht reflektieren, eine spiegelnde Parabel (5) enthalten, angeordnet in Längsrichtung und in dem vom Boden eines rohrförmigen Kanals (C) angehobenen Teil, welcher den hohlen Teil des Handgriffes (M), der die genannte Lichtquelle (F) trägt, mit dem äusseren Ende des Retraktorrohres verbindet, und der im Inneren des genannten Rohres mündet.

3. Vorrichtung nach Patentanspruch 2, bei welcher der rohrförmige Kanal (C), der die lichtreflektierende Parabel (5) aufnimmt, wenigstens aussen eine flache Konfiguration hat, welche von einer entsprechenden Verstärkung der Seiten kommt, und zwar auf solche Weise, dass er sehr widerstandsfähig gegen Durchbiegung und Verdrehung ist.

4. Vorrichtung nach Patentanspruch 2, bei welcher wenigstens der obere Abschnitt (601) des Kanals (C), welcher die reflektierende Parabel (5) aufnimmt, auf den anderen Teil desselben Kanals aufgesetzt und an diesem befestigt ist, mit dem Zweck, die Realisierung und/oder Installierung der genannten reflektierenden Parabel zu erleichtern.

5. Vorrichtung nach Patentanspruch 4, bei welcher die reflektierende Parabel (5) mit einem elektro-chemischen Verfahren der Verchromung der inneren Oberfläche des oberen und aufgesetzten Abschnittes (601) des Kanals (C) realisiert ist.

6. Vorrichtung nach Patentanspruch 4, bei welcher der obere und aufgesetzte Abschnitt (601) des Kanals (C) in einem Stück und mit einer entsprechend angeschlossenen Verbindung mit dem oberen, schalenförmigen Abschnitt (501') des Handgriffes (M) hergestellt ist, wobei der genannte Abschnitt zum Beispiel für eine Schnappverbindung vorgesehen ist, und zwar mit Hilfe von Steck- und Aufnahmeansätzen (2, 2'), und mit dem ergänzenden unteren Abschnitt ebenfalls schalenförmig (501), welcher den Handgriff (M) vervollständigt, der fest mit dem in Längsrichtung verrippten und nach aussen hin konvexen Abschnitt (401) verbunden ist, welcher den unteren Abschnitt desselben Kanals (C) bildet, und welcher in einem Ansatz (301) von einem im wesentlichen dreieckigen Umriss erhalten ist, und zwar mit Seiten, die tangential zu dem äusseren Rand der konischen Öffnung (201) des Retraktorrohres verlaufen.

7. Vorrichtung nach Patentanspruch 6, bei welcher der Anschlussbereich des oberen Abschnittes (601) des Kanals (C) mit der internen lichtreflektierenden Parabel (5) an die obere Schale (501') zum Bilden des Handgriffes (M) eine zu dem Handgriff hin angehobene Stufe (11) aufweist, an welche der Benutzer vorteilhafterweise den Daumen der Hand anlegen kann, mit welcher er den Handgriff selbst ergreift, um einen festeren Halt und eine leichtere Benutzung der Vorrichtung zu sichern.

8. Vorrichtung nach Patentanspruch 6, bei welcher in dem Abschnitt, mit dem die beiden Schalen (501, 501') zum Bilden des Handgriffes (M) an die Abschnitte (601, 401) des Kanals (C) angeschlossen sind, der die lichtreflektierende Parabel (5) enthält, in den genannten Schalen ergänzend Abschnitte zum Bilden eines Sitzes (3) erhalten sind, in welchen es möglich ist, eine kleine Scheibe (4) aus transparentem Material einzusetzen, welche in so gut wie möglich abdichtender Weise den genannten Kanal (C) von dem Sitz des Handgriffes trennt, in welchem die durch Reibung oder auf andere lösbare Weise gehaltene Leuchtquelle (F) angeordnet ist.

9. Vorrichtung nach Patentanspruch 8, bei welcher die kleine transparente Scheibe (4) optische Funktionen haben kann, zum Beispiel kann sie aus einer Linse gemacht sein, welche das von der Leuchtquelle (F) kommende Licht auf der lichtreflektierenden Parabel (5) fokalisiert.

10. Vorrichtung nach den vorstehenden Patentansprüchen, bei welcher der Kanal (C) mit der lichtreflektierenden Parabel (5) eine Neigung zwischen 30° und 50°, zum Beispiel 43°, im Verhältnis zu der Achse des Retraktorrohres (1) aufweist, während der zwischen dem Handgriff (M) und der Achse des Retraktorrohres (1) vorhandene interne Winkel zwischen 100° und 120° beträgt, zum Beispiel um 110°.

11. Vorrichtung nach den vorstehenden Patentansprüchen, **gekennzeichnet durch** die Tatsache, dass sie aus einem beliebigen geeigneten Kunststoffmaterial von changierender weisser Farbe hergestellt ist, welches die interne Beleuchtung des Retraktorrohres (1) und insbesondere von dessen seitlichem Fenster (8) begünstigt.

12. Vorrichtung nach einem oder mehreren der Patentansprüche von 2 bis 11, **gekennzeichnet durch** die Tatsache, dass an dem unteren Abschnitt (401) des Kanals (C), welcher die lichtreflektierende Parabel (5) enthält, wenigstens eine Öffnung (6) zum Ablassen nach aussen von möglicher organischer Flüssigkeit vorgesehen sein kann, die durch Schwerkraft an die genannte Öffnung gelangt, und um zu vermeiden, dass solche Flüssigkeit bis an die kleine Scheibe (4) kommt, die stromaufwärts der Leuchtquelle (F) angeordnet ist.

13. Vorrichtung nach einem oder mehreren der Patentansprüche von 2 bis 12, bei welcher der Kanal (C), der die lichtreflektierende Parabel (5) enthält, entgegengesetzt zu derselben Parabel einen breiten vertieften Bereich (14) aufweist, welcher einen Abschnitt des Endes der optischen Beleuchtungsfaser (F) frei lässt, angeordnet in kurzem Abstand von der genannten Parabel.

14. Vorrichtung nach Patentannspruch 1, bei welcher das Fenster (8) zum Untersuchen der Analschleimhaut auf einer idealen Ebene liegt, welche im wesentlichen parallel zu der mittleren Ebene derselben Vorrichtung verläuft und rechts oder links von der Retraktorsonde angeordnet sein kann, wenn dieselbe Vorrichtung mit dem Handgriff (M) nach unten gerichtet betrachtet wird.

15. Vorrichtung nach Patentannspruch 1, bei welcher das Fenster (8) zum Untersuchen der Analschleimhaut auf einer idealen Ebene liegt, welche im wesentlichen lotrecht zu der mittleren Ebene derselben Vorrichtung verläuft und oberhalb angeordnet ist, wenn dieselbe Vorrichtung mit dem Handgriff (M) nach unten gerichtet betrachtet wird.

16. Vorrichtung nach Patentannspruch 1, bei welcher das Fenster (8) zum Untersuchen der Analschleimhaut einen Abstand von dem Ende des Retraktorrohres hat, das an das konische Ende (201) angeschlossen ist, der zwischen 4 und 7 Zentimeter beträgt, zum Beispiel um 5-6 Zentimeter.

17. Vorrichtung nach Patentannspruch 1, bei welcher das Fenster (8) zum Untersuchen der Analschleimhaut in einem abgeflachten und leicht vertieften Abschnitt (701) der Seitenwand des Retraktorrohres (1) liegt, welcher sich dicht an dem abgerundeten und geschlossenen Ende (101) des genannten Rohres befindet, wobei die hintere Seite (208) des genannten Fensters abgerundet mit einer geneigten Ebene (801) mit der seitlichen Oberfläche des genannten Rohres verbunden ist, während die vordere Seite (108) desselben Fensters ebenfalls abgerundet und entsprechend angehoben ist und eine gebogene Form aufweist, um insgesamt eine bessere Anordnung des Analgewebes zur Untersuchung und für den Eingriff durch das genannte Fenster zu haben.

18. Vorrichtung nach Patentannspruch 1, bei welcher das Fenster (8) zum Untersuchen der Analschleimhaut quer in das Retraktorrohr eingearbeitet ist und in demselben um etwa die Hälfte seines Umfangs verläuft, wobei die hintere Seite (208) des genannten Fensters angerundet und mit Hilfe einer breiten geneigten Ebene (801) mit der seitlichen Oberfläche des genannten Rohres verbunden ist, während die ebenfalls abgerundete vordere Seite (108) desselben Fensters im Verhältnis zu der genannten hinteren Seite entsprechend abgesenkt und an flache Abschnitte (701', 701) angeschlossen ist, welche sich sinusförmig und mit einem abnehmenden Profil zu der abgerundeten Spitze (101) des Retraktorrohres hin erstrecken.

19. Vorrichtung nach Patentanspruch 1, **gekennzeichnet durch** die Tatsache, dass im Inneren des Retraktorrohres (1) unter dem seitlichen Fenster (8) zum Untersuchen der Analschleimhaut, mit einem kurzen Abstand und vorzugsweise auf der Ebene der Mittellinie, Mittel (12) vorgesehen sind, geeignet zum Aufnehmen und zum drehbaren Zentrieren des Endes einer Spindel, welche die gebogene Nadel für die Ligatur der Arteria haemorrhoidalis trägt.

20. Vorrichtung nach Patentanspruch 19, bei welcher die genannten Aufnahme- und Zentriermittel (12) aus einem Sitz mit rundem Querschnitt gebildet sind, angeordnet mit seiner Achse parallel zu und mit einem korrekten Abstand von der Achse des Retraktorrohres (1).

21. Vorrichtung nach Patentanspruch 20, bei welcher der genannte Sitz (12) eine konische Form hat und zu der Spitze des Retraktorrohres (1) hin schmaler wird.

22. Vorrichtung nach Patentanspruch 1, bei welcher die seitliche Öffnung (7), durch welche sich der empfindliche Teil der Ultraschallsonde (S) erstreckt, den geneigten Abschnitt (801) betrifft, welcher an der hinteren Wand (208) des Fensters (8) zum Untersuchen der Analschleimhaut konvergierend ist.

23. Vorrichtung nach Patentanspruch 1, bei welcher die Ultraschallsonde (S) hygienisch durch eine sterile, leicht zu entsorgende und entfernbare Hülle geschützt sein kann, um eine hygienische Wiederverwendung derselben Sonde zu erlauben.

24. Vorrichtung nach einem oder mehreren der vorstehenden Patentansprüche, bei welcher die Seite der konischen Öffnung (201) des Retraktorrohres auf der Seite des Sitzes (10) zur Aufnahme der Ultraschallsonde (S) abgeflacht ist und Ansätze (13) trägt, welche die Funktion von Schlaufen haben, an denen es möglich ist, das Kabel (G) der Sonde selbst lösbar zu verankern.

25. Vorrichtung nach Patentanspruch 24, bei welcher der Handgriff (M) auf einer Seite und dicht an der Ultraschallsonde (S) mit Ansätzen versehen sein kann, welche kleine Schlaufen formen, an denen es möglich ist, einen weiteren Abschnitt des mehrpoligen Kabels, das an die genannte Ultraschallsonde (S) angeschlossen ist, lösbar zu verankern.

## Revendications

1. Dispositif jetable pour des opérations chirurgicales sur l'artère hémorroïdale, comprenant un tube écarteur (1) fermé et arrondi sur l'extrémité avec laquelle il est inséré dans l'orifice anal, qui est pourvu sur sa surface latérale d'au moins une fenêtre (8) au travers de laquelle apparaît la muqueuse rectale sur laquelle l'opération de ligature de l'artère sera pratiquée, qui est pourvu d'une poignée de préhension (M) faisant partie intégrante de sa bouche externe et préférablement divergente (201), au travers de laquelle il est possible d'observer le tissu au travers de ladite fenêtre et il est possible d'insérer dans le tube écarteur (1) les instruments requis pour l'opération chirurgicale, comprenant des moyens d'illumination de ladite fenêtre et de la muqueuse qui apparaît au travers de cette dernière, **caractérisé en ce que** lesdits moyens d'illumination comprennent une source lumineuse (F) fixée de manière amovible à l'intérieur de ladite poignée de préhension (M) et comprennent au niveau de la zone de conjonction de ladite poignée de préhension (M) à l'extrémité externe du tube écarteur (1) des moyens (5) pour réfléchir la lumière fournie par ladite source lumineuse (F) et pour permettre que ladite lumière réfléchie illumine la partie interne du tube écarteur (1) et en particulier ladite fenêtre latérale (8) ; le tube écarteur (1) étant prévu en alignement avec la fenêtre (8) et, en amont de cette dernière, avec une petite chambre (10) longitudinale, rectiligne et interne, délimitée par une cloison (9) faisant partie intégrante des parois internes du tube écarteur (1), étant prévu que dans ladite chambre (10) est logée une sonde à ultrasons (S) partiellement saillante au travers d'une ouverture (7) formée longitudinalement sur la paroi latérale du tube écarteur (1) de manière à ce que cette sonde à ultrasons (S) soit en contact avec la muqueuse anale.

2. Dispositif selon la revendication 1, **caractérisé en ce que** les moyens qui reflètent la lumière fournie par la source lumineuse (F) comprennent une parabole spéculaire (5) placée longitudinalement et dans la partie soulevée du fond d'un canal tubulaire (C) qui relie la partie vide de la poignée (M), qui accueille ladite source lumineuse (F), à l'extrémité externe du tube écarteur et qui finit à l'intérieur dudit tube.

3. Dispositif selon la revendication 2, dans lequel le canal tubulaire (C), qui loge la parabole de réflexion (5) de la lumière, présente au moins extérieurement une configuration plate qui dérive d'un renforcement adapté des côtés, de manière à être très résistant en flexion et torsion.

4. Dispositif selon la revendication 2, dans lequel au moins la portion supérieure (601) du canal (C) qui accueille la parabole de réflexion (5) est insérée et fixée sur l'autre portion du même canal avec l'objectif de simplifier la réalisation et/ou l'installation de ladite parabole de réflexion.

5. Dispositif selon la revendication 4, dans lequel la parabole de réflexion (5) est réalisée grâce à un procédé électrochimique de chromage de la surface interne de la portion supérieure et insérée (601) du canal (C).

6. Dispositif selon la revendication 4, dans lequel la portion supérieure et insérée (601) du canal (C) avec la parabole (5) de réflexion de la lumière est intégralement obtenue et avec une connexion opportunément reliée, avec la portion en forme de coquille (501') de la poignée de préhension (M), ladite portion étant prédisposée par exemple pour l'accouplement par encliquetage, au moyen d'appendices mâle-femelle (2, 2'), avec la complémentaire portion inférieure en forme de coquille (501) qui complète la poignée (M) et qui fait partie intégrante de la portion convexe vers l'extérieur et longitudinalement nervurée (401) qui forme la partie inférieure du même canal (C) et qui est obtenue dans un appendice (301) ayant un plan substantiellement triangulaire, avec des côtés qui sont tangents au bord externe de la bouche conique (201) du tube écarteur.

7. Dispositif selon la revendication 6, dans lequel la zone de connexion de la portion supérieure (601) du canal (C) avec la parabole de réflexion de la lumière interne (5) à la coquille supérieure (501') pour la formation de la poignée (M) présente une surélévation (11) vers ladite poignée, sur laquelle l'opérateur peut avantageusement reposer le pouce de la main qui prend ladite poignée, pour assurer une prise plus sure et une utilisation facilitée du dispositif.

8. Dispositif selon la revendication 6, dans lequel, dans la portion avec laquelle les deux coquilles (501, 501') pour la formation de la poignée (M) sont connectées avec les portions (601, 401) du canal (C) qui contient la parabole de réflexion de la lumière (5) en amont dudit canal, sont obtenues dans lesdites coquilles des portions complémentaires pour la formation d'un logement (3) dans lequel il est possible de loger un petit disque (4) en matériau transparent, qui divise le plus possible de manière étanche ledit canal (C) du logement de la poignée dans laquelle est fixée par frottement ou de toute autre manière amovible la source de lumière (F).

9. Dispositif selon la revendication 8, dans lequel le petit disque transparent (4) peut avoir des fonctions optiques, en étant par exemple une lentille qui focalise sur la parabole de réflexion (5) la lumière fournie par la source lumineuse (F).

10. Dispositif selon les revendications précédentes, dans lequel le canal (C) avec la parabole de réflexion de la lumière (5) présente une inclinaison comprise entre 30° et 50°, par exemple 43°, par rapport à l'axe du tube écarteur (1), alors que l'angle interne existant entre la poignée (M) et l'axe du tube écarteur (1) est compris entre 100° et 120° et est par exemple de 110°.

11. Dispositif selon les revendications précédentes, **caractérisé en ce qu'**il est formé de n'importe quelle matière plastique adaptée de couleur blanche changeante, qui facilite l'illumination interne du tube écarteur (1) et en particulier de sa fenêtre latérale (8).

12. Dispositif selon une ou plusieurs des revendications 2 à 11, **caractérisé en ce que** sur la portion inférieure (401) du canal (C) qui contient la parabole de réflexion de la lumière (5) peut être prévue au moins une ouverture (6) pour évacuer vers l'extérieur le possible liquide organique qui arrive par gravité à ladite ouverture et pour empêcher que ce liquide n'arrive sur le petit disque (4) placé vers le haut de la source lumineuse (F).

13. Dispositif selon une ou plusieurs des revendications 2 à 12, dans lequel le canal (C) qui contient la parabole de réflexion de la lumière (5) présente en contraste avec cette parabole une large zone en renfoncement (14) qui laisse découverte une portion de l'extrémité de la fibre optique d'illumination (F) placée à brève distance de ladite parabole.

14. Dispositif selon la revendication 1, dans lequel la fenêtre (8) d'exploration de la muqueuse anale est comprise dans un plan idéal substantiellement parallèle au plan central du même dispositif et peut être placée sur la droite ou la gauche de la sonde de l'écarteur, si le même dispositif est considéré avec la poignée (M) orientée vers le bas.

15. Dispositif selon la revendication 1, dans lequel la fenêtre (8) pour l'exploration de la muqueuse anale est comprise dans un plan idéal substantiellement perpendiculaire au plan central du même dispositif et est placée vers le haut si le dispositif est considéré avec la poignée (M) orientée vers le bas.

16. Dispositif selon la revendication 1, dans lequel la fenêtre (5) pour l'exploration de la muqueuse anale présente une distance par rapport à l'extrémité du tube écarteur, connectée à l'extrémité conique (201), comprise entre 4 et 7 centimètres, par exemple d'environ 5-6 centimètres.

17. Dispositif selon la revendication 1, dans lequel la fenêtre (8) pour l'exploration de la muqueuse anale est comprise dans une portion en léger renfoncement aplatie (701) de la paroi latérale du tube écarteur (1) proche de l'extrémité fermée arrondie (101) dudit tube, le côté postérieur (208) de ladite fenêtre étant arrondi et connecté avec un plan incliné (801) à la surface latérale dudit tube, alors que le côté antérieur (108) de la même fenêtre est également arrondi et opportunément soulevé et présente une forme arquée, le tout pour une meilleure disposition du tissu anal pour l'exploration et pour l'opération au travers de ladite fenêtre.

18. Dispositif selon la revendication 1, dans lequel la fenêtre (8) pour l'exploration de la muqueuse anale est obtenue transversalement sur le tube écarteur et intéresse ce dernier sur environ la moitié de sa circonférence, le côté postérieur (208) de ladite fenêtre étant arrondi et connecté au moyen d'un large plan incliné (801) à la surface latérale dudit tube, alors que le côté antérieur (108) de la même fenêtre est également arrondi, est opportunément abaissé par rapport audit côté postérieur et est connecté à des portions plates (701, 701') qui s'étendent avec une forme sinueuse et avec un profil décroissant vers le point arrondi (101) du tube écarteur.

19. Dispositif selon la revendication 1, **caractérisé en ce qu'**à l'intérieur du tube écarteur (1) sous la fenêtre latérale (8) pour l'exploration de la muqueuse anale, à brève distance et préférablement au niveau de sa ligne centrale, sont prévus des moyens (12) adaptés à recevoir et à centrer en rotation l'extrémité d'un mandrin qui supporte l'aiguille courbée pour la ligature de l'artère hémorroïdale.

20. Dispositif selon la revendication 19, dans lequel lesdits moyens d'accueil et de centrage (12) sont constitués d'un logement à section arrondie, placé avec son axe parallèle et avec une distance correcte par rapport à l'axe du tube écarteur (1).

21. Dispositif selon la revendication 20, dans lequel ledit logement (12) présente une forme conique et se rétrécit vers le point du tube écarteur (1).

22. Dispositif selon la revendication 1, dans lequel l'ouverture latérale (7) au travers de laquelle est saillante la portion sensible de la sonde à ultrasons (S) intéresse la portion inclinée (801) convergente sur la paroi postérieure (208) de la fenêtre (8) pour l'exploration de la muqueuse anale.

23. Dispositif selon la revendication 1, dans lequel la sonde à ultrasons (S) peut être hygiéniquement protégée dans une gaine stérile, jetable et facilement amovible, pour permettre une réutilisation hygiénique de la sonde.

24. Dispositif selon une ou plusieurs des revendications précédentes, dans lequel le côté de la bouche conique (201) du tube écarteur est aplati sur le côté du logement (10) pour le logement de la sonde à ultrasons (S) et porte des appendices (13) ayant la fonction d'organes passants auxquels il est possible d'accrocher de manière amovible le câble (G) de ladite sonde.

25. Dispositif selon la revendication 24, dans lequel la poignée (M) peut être pourvue sur le côté à proximité de la sonde à ultrasons (S) d'appendices formant de petits organes passants auxquels peut être ancrée de manière amovible une autre portion du câble multipolaire connecté à ladite sonde à ultrasons (S).
